# FASCICULE DE BREVET EUROPEEN

(11) **EP 0 773 175 B1**
(45) Date de publication et mention de la délivrance du brevet: **19.01.2000**
(21) Numéro de dépôt: 96420321.0
(22) Date de dépôt: 30.10.1996
(51) Int. Cl.: B65F 1/10, B65F 1/14, B65F 7/00, A61B 19/02

(54) **Collecteur de déchets spécifiques, notamment médicaux**
Sammelbehälter für spezifische Abfälle,insbesondere medizinische Abfälle
Collecting container for specific waste, especially medical waste

(30) Priorité: 08.11.1995 FR 9513437
(43) Date de publication de la demande: 14.05.1997
(73) Titulaire: Zenon, 75116 Paris (FR)
(72) Inventeur: Blandin, Claude, 38360 Sassenage (FR)
(74) Mandataire: Maureau, Philippe

(56) Documents cités:
- EP-A- 0 543 763
- FR-A- 2 705 952
- FR-A- 2 706 431
- FR-A- 2 714 896

## Description

La présente invention concerne un collecteur de déchets spécifiques, notamment médicaux.

Les déchets présentant des risques pour la collectivité, soit par leur caractère toxique ou contaminant, soit par la prolifération bactérienne à laquelle ils peuvent donner lieu, font l'objet depuis quelques années d'une collecte par des moyens spéciaux.

Le document FR-A-2 714 896 décrit un collecteur adapté à de tels déchets. Ce collecteur comprend une enceinte en béton, dans laquelle sont placés un réceptacle intermédiaire de dépôt provisoire des déchets et un conteneur de stockage de ces déchets. Le réceptacle intermédiaire est délimité pour partie par un tiroir coulissant qui déplace les déchets depuis une position de réception, dans laquelle il communique avec l'extérieur du collecteur, vers une position de déversement dans un conteneur.

Le mouvement du réceptacle intermédiaire est commandé par une carte magnétique ou à microprocesseur, que détient l'utilisateur habilité.

Ce collecteur peut être équipé d'une unité d'identification du type d'emballage dans lequel sont placés les déchets, d'une unité de désinfection dudit emballage, d'une unité de congélation des déchets dans le réceptacle intermédiaire ou dans le conteneur.

Il est apparu que ce collecteur pouvait être perfectionné de manière à faciliter la collecte et la gestion des déchets, et à renforcer encore les garanties de sécurité au plan sanitaire.

La présente invention vise à fournir un collecteur atteignant ces objectifs.

Le collecteur qu'elle concerne comprend une enceinte en béton, un réceptacle intermédiaire, un conteneur amovible de stockage des déchets comprenant un couvercle muni d'une trappe normalement fermée, escamotable par coulissement, et des moyens de mise en communication du réceptacle intermédiaire alternativement avec l'extérieur du collecteur et avec le conteneur, tels que précités.

Selon l'invention, le réceptacle intermédiaire est disposé au-dessus du conteneur de réception des déchets et est normalement fermé, en partie inférieure, par une trappe escamotable par coulissement située au-dessus du couvercle du conteneur, ladite trappe que comprend le couvercle du conteneur étant escamotable selon la même direction que la trappe du réceptacle intermédiaire, au moins l'une de ces trappes étant munie de moyens permettant la venue en prise des deux trappes l'une avec l'autre lors de leur ouverture, de sorte que la trappe du réceptacle intermédiaire, lorsqu'elle est déplacée dans le sens de son ouverture, déplace la trappe du couvercle du conteneur dans le sens de l'ouverture.

Ainsi, le réceptacle intermédiaire n'est pas coulissant entre une position de réception des déchets et une position de déversement, mais est monté de manière fixe dans le collecteur. Il en résulte une plus grande sécurité au niveau du déversement, avec réduction du risque de blocage ou de coincement, et une parfaite garantie sur un plan sanitaire, du fait de la présence des deux trappes. En outre, ce montage permet un gain d'encombrement important.

De préférence, le collecteur comprend un compartiment fermé, isolé thermiquement, dans lequel est placé le conteneur de stockage des déchets, ainsi qu'une centrale de production de froid à l'intérieur de ce compartiment, la trappe du réceptacle intermédiaire étant elle-même isolée thermiquement et assurant la fermeture de ce compartiment isolé.

Ce compartiment constitue, avec l'enceinte en béton et le conteneur, une troisième enveloppe, parfaitement étanche, de confinement des déchets, et permet une conservation de ces déchets à une température basse, de l'ordre de 6°C, inhibant toute prolifération microbienne. Le collecteur selon l'invention assure de cette manière une totale sécurité sur le plan sanitaire.

De préférence, les différents organes du collecteur, à l'exception du conteneur et de l'éventuel compartiment isolé, ou une partie de ces organes seulement, sont montés sur un châssis coulissant disposé au-dessus du conteneur et de l'éventuel compartiment isolé, qui peut être au moins partiellement extrait du collecteur.

Ce montage permet de faciliter l'entretien des organes qui seraient, autrement, inaccessibles ou difficilement accessibles. De plus, l'éloignement de ces organes grâce à ce châssis permet un nettoyage à grande eau et à tout moment de l'emplacement du conteneur, en particulier du compartiment isolé précité.

Avantageusement, le réceptacle intermédiaire comprend un télémètre dont le signal est dirigé vers l'intérieur du conteneur. Ce télémètre permet, lorsque les trappes sont ouvertes, de déterminer le niveau de remplissage du conteneur et donne une information sur la nécessité de vider ce dernier.

Selon d'autres caractéristiques avantageuses, pouvant être prévues alternativement ou cumulativement, le collecteur conforme à l'invention comprend :
- une goulotte de section carrée pour l'introduction des déchets, délimitée par quatre pans latéraux qui convergent vers l'intérieur du collecteur, afin de faciliter cette introduction ;
- un système photo-électrique placé au niveau de cette ouverture, relié aux moyens de commande de l'ouverture et de la fermeture de la porte coulissante venant obturer le réceptacle intermédiaire vis-à-vis de l'extérieur, ce système permettant d'interdire la fermeture de cette porte en cas de présence d'un obstacle en travers de l'ouverture, tel que les mains de l'opérateur ou un carton de déchet mal engagé ;
- un lecteur de carte muni d'un volet de recouvrement de la fente d'introduction de la carte et de moyens de verrouillage de ce volet en position abaissée au-dessus de la fente, ce volet et ces moyens de verrouillage étant reliés aux moyens de commande de la porte coulissante du réceptacle intermédiaire ; l'ensemble permet, lorsque le volet est déplacé après que la carte ait été introduite dans le lecteur, le déclenchement de l'ouverture de la porte coulissante du réceptacle intermédiaire et le verrouillage du volet en position abaissée tant que la porte n'est pas refermée ; il oblige l'utilisateur à attendre la fermeture complète de la porte pour récupérer sa carte, et l'incite à ne pas laisser le sac de déchets dans le cas où la porte ne se fermerait pas ;
- un ensemble de pulvérisation de produit aseptique sur les déchets, lorsque ceux-ci se trouvent dans le réceptacle intermédiaire ;
- une bavette frottant sur le dessus de la trappe escamotable du réceptacle intermédiaire, pour assurer le nettoyage de cette dernière et, le cas échéant, évacuer l'excès de produit aseptique ;
- un système de pesage encastré dans la trappe du réceptacle intermédiaire, permettant de déterminer le poids de déchets déposés en vue d'imputer à l'utilisateur correspondant des frais de collecte et d'incinération proportionnels ;
- un peigne ou similaire fixé à la trappe escamotable du conteneur, permettant, lors du mouvement de cette trappe, de brasser les déchets afin de les répartir sur le fond du conteneur ;
- deux rampes permettant de faciliter l'introduction et le retrait du conteneur de déchets, qui est monté sur roulettes, dans le collecteur ou, le cas échéant, dans le compartiment isolé, ces rampes étant basculables dans une position de repliage, dans laquelle elles portent contre la paroi latérale du conteneur et maintiennent celui-ci en position de remplissage, sous le réceptacle intermédiaire ;
- un dispositif modulateur/démodulateur, dit "modem", de transmission vers un centre de gestion des données recueillies à partir de la carte de l'utilisateur, ainsi que, le cas échéant, à partir du télémètre ou du dispositif de pesage ; ce dispositif permet de programmer les interventions de vidage du conteneur ainsi que les interventions de maintenance du collecteur ;
- une enceinte en béton coulé en une seule pièce, assurant une étanchéité parfaite de l'ensemble ;
- deux portes arrière en acier, munies de serrures à trois points d'ancrage et d'un détecteur de leur position de fermeture ;
- une alarme sonore anti-vandalisme, à déclenchement en cas de violation des portes de fermeture du collecteur.

Pour sa bonne compréhension, l'invention est à nouveau décrite ci-dessous en référence au dessin schématique annexé représentant, à titre d'exemple non limitatif, une forme de réalisation préférée du collecteur qu'elle concerne.
La figure 1 en est une vue en perspective, avec arrachement ;
la figure 2 en est une vue en coupe longitudinale, en élévation, et
la figure 3 en est une vue en élévation, de l'arrière, selon une variante de réalisation.

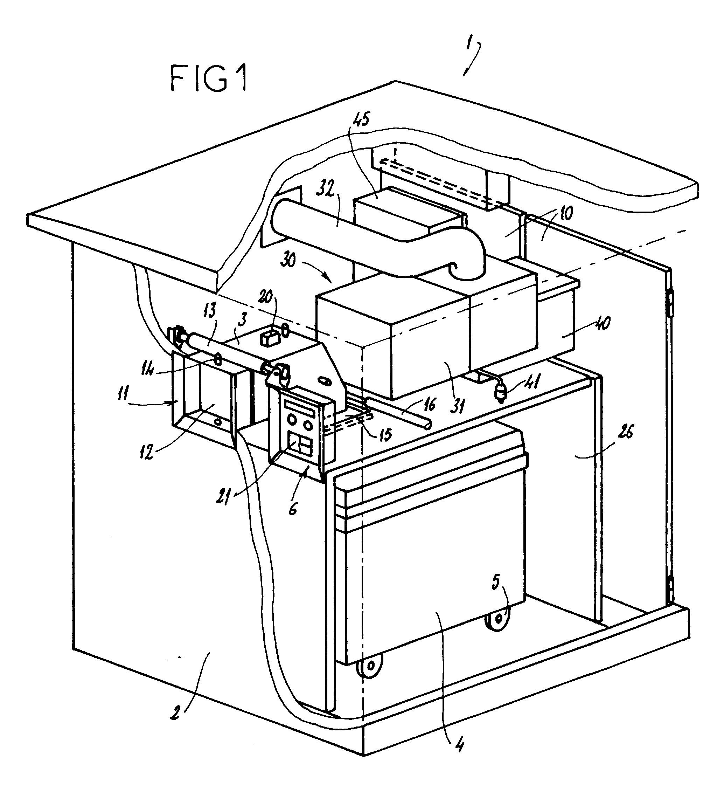

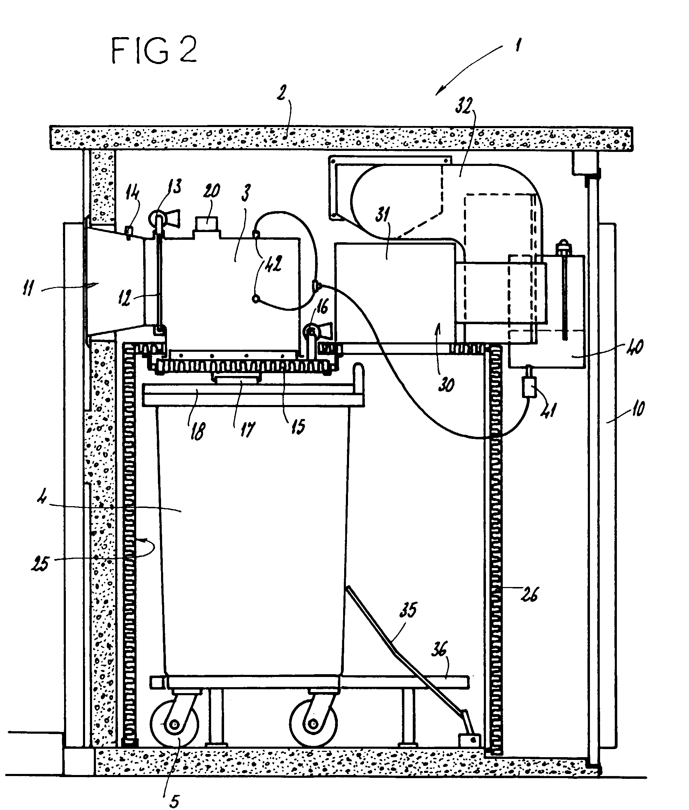

Les figures 1 et 2 représentent, sous différents angles, un collecteur 1 de déchets spécifiques, notamment médicaux.

Ce collecteur 1 comprend une enceinte 2 en béton coulé en une seule pièce, dans laquelle sont placés un réceptacle intermédiaire 3 de dépôt provisoire des déchets, un conteneur 4 de stockage de ces déchets, monté sur des roulettes 5, un lecteur 6 de carte magnétique ou à microprocesseur, et divers autres organes décrits plus loin.

Pour la clarté des figures, les conduites de connexion de ces différents organes du collecteur 1 ne sont par représentées.

L'enceinte 2 est fermée à une extrémité par deux portes 10 en acier, munies de serrures à trois points d'ancrage et d'un détecteur de position de fermeture des portes (non représentés).

Le réceptacle intermédiaire 3 est disposé au-dessus du conteneur 4, et est fixe en position.

Il est constitué par un caisson ouvert latéralement à son extrémité opposée aux portes 10, et ouvert en partie inférieure.

L'ouverture latérale permet au réceptacle intermédiaire 3 de communiquer avec une goulotte 11 de section carrée, délimitée par quatre pans latéraux qui convergent vers l'intérieur du collecteur 1. Cette goulotte 11 est fermée par une porte coulissante 12 actionnée par un vérin 13. Elle comprend également un émetteur et un récepteur photo-électriques 14 disposés verticalement au niveau de l'ouverture d'introduction des déchets, qui sont reliés à l'unité de commande du vérin 13, et qui empêchent tout actionnement de la porte 12 lorsque le faisceau lumineux est interrompu.

L'ouverture inférieure du réceptacle intermédiaire 3 permet à ce réceptacle de communiquer avec le conteneur 4. Cette ouverture est normalement fermée par une trappe 15, escamotable par coulissement, actionnée par un vérin 16 selon une direction perpendiculaire à la direction d'introduction des déchets. Cette trappe 15 est isolée thermiquement et est munie d'une saillie inférieure 17 apte à venir en prise avec une saillie complémentaire aménagée sur une trappe 18 que comprend le couvercle du conteneur 4. Cette dernière est mobile en coulissement selon la même direction que la trappe 15, et est rappelée en position de fermeture par des moyens électriques appropriés, tels que des vérins.

Le réceptacle intermédiaire 3 comprend en outre un télémètre 20 dont le signal est dirigé vers l'intérieur du conteneur 4.

Le lecteur 6 est muni d'un volet 21 de recouvrement de la fente d'introduction de la carte et des moyens pour le verrouillage de ce volet en position abaissée, ce volet et ces moyens de verrouillage étant reliés fonctionnellement à l'unité de commande du vérin 13. L'ensemble est prévu de manière à ce que ce volet 21, lorsqu'il est déplacé après que la carte ait été introduite dans le lecteur 6, permette le déclenchement de l'ouverture de la porte 12, et à ce que le volet 21 soit verrouillé en position abaissée tant que la porte 12 n'est pas refermée.

Ainsi que cela apparaît aux figures 1 et 2, le conteneur 4 est placé dans un compartiment 25, isolé thermiquement, qui est fermé par la trappe 15 en partie supérieure et par une porte 26 du côté des portes 10. Cette porte 26 permet l'introduction et le retrait du conteneur 4.

Le compartiment 25 est relié à une centrale 30 de production de froid, comprenant une unité de réfrigération 31 et une conduite 32 d'évacuation de l'air chaud, débouchant sur l'extérieur du collecteur 4.

Le compartiment 25 comprend en outre :
- deux rampes 35 basculables par pivotement entre la position de repliage représentée, dans laquelle elles portent contre la paroi latérale du conteneur 4 et maintiennent celui-ci en position de remplissage, sous le réceptacle intermédiaire 3, et une position d'introduction/retrait du conteneur 4, dans laquelle elles s'étendent près du sol, jusqu'au-delà des portes 10, et
- des glissières latérales 36 facilitant le déplacement du conteneur 4 dans le compartiment 25.

Le collecteur 1 est également muni d'un ensemble de pulvérisation de produit aseptique sur les déchets, lorsque ceux-ci se trouvent dans le réceptacle intermédiaire 3. Cet ensemble comprend un réservoir 40 de produit aseptique, une pompe 41 et trois buses de pulvérisation 42, montées sur la paroi supérieure et les parois latérales du réceptacle intermédiaire 3. Une bavette (non représentée) frottant sur le dessus de la trappe 15 peut être prévue sur le réceptacle 3 pour assurer le nettoyage de cette trappe et, le cas échéant, évacuer l'excès de produit aseptique.

Le collecteur 1 comprend en outre un boîtier 45, abritant les connexions électriques et un dispositif modulateur/démodulateur dit "modem". Ce modem permet la transmission vers un centre de gestion des données recueillies à partir de la carte de l'utilisateur et du télémètre ou d'un éventuel dispositif de pesage intégré à la trappe 15.

En pratique, l'utilisateur introduit sa carte dans le lecteur 6 et abaisse le volet 21, ce qui permet l'ouverture de la porte 12. Après introduction des déchets, la porte 12 se referme, à condition que le faisceau lumineux entre l'émetteur et le récepteur photo-électriques 14 ne soit pas interrompu. L'utilisateur peut récupérer sa carte après fermeture complète de la porte 12, qui provoque le déverrouillage du volet 21.

Après un éventuel pesage et une aspersion de produit aseptique, la trappe 15 s'escamote, entraînant avec elle la trappe 18. Les déchets tombent dans le conteneur 4, tandis que le télémètre 20 mesure, au moment de l'ouverture des trappes 15 et 18, la hauteur des déchets dans le conteneur 4. Les trappes 15 et 18 se referment ensuite.

L'information fournie par le télémètre 20, ainsi que celles résultant de la lecture de la carte et du pesage, est transmise par le modem vers le centre de gestion, ce qui permet notamment de programmer les interventions de vidage du conteneur ainsi que les interventions de maintenance du collecteur.

Le compartiment 25 constitue, avec l'enceinte 2 et le conteneur 4, une troisième enveloppe, parfaitement étanche, de confinement des déchets, et permet une conservation de ces déchets à une température basse, de l'ordre de 6°C, inhibant toute prolifération microbienne. Le collecteur 1 selon l'invention assure de cette manière une parfaite sécurité sur le plan sanitaire.

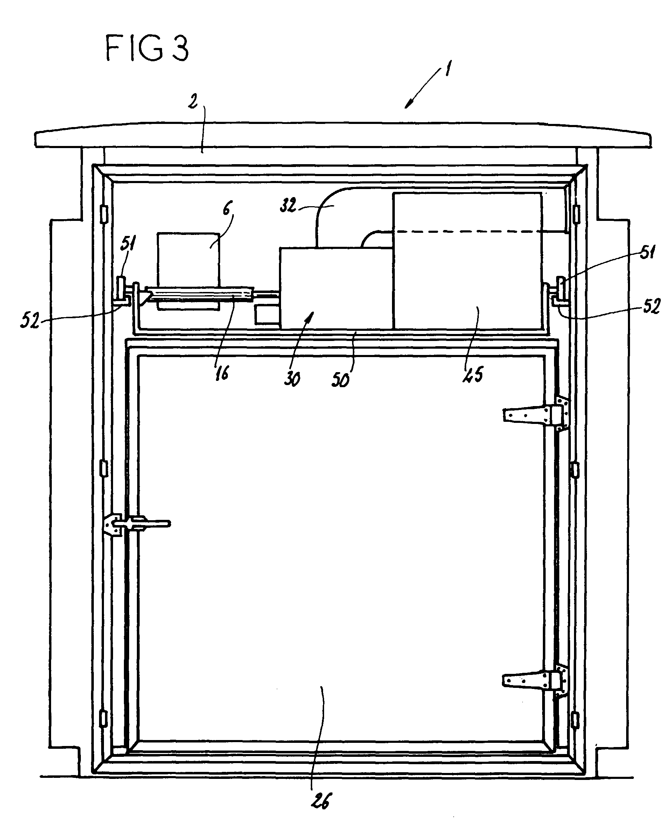

La figure 3 montre une variante de réalisation du collecteur 1. Par simplification, les éléments déjà décrits en référence aux figures 1 et 2 qui se retrouvent dans cette variante sont désignés par les mêmes références numériques.

Il apparaît que dans ce cas les différents organes du collecteur 1, à l'exception du conteneur 4 et du compartiment isolé 25, sont montés sur un châssis 50 disposé au-dessus du compartiment isolé 25, ce châssis étant muni de galets latéraux 51, qui coulissent dans des glissières 52 fixées aux parois latérales de l'enceinte 2.

Le châssis 50 peut être au moins partiellement extrait du collecteur 1, ce qui permet de faciliter l'entretien des différents organes et de nettoyer à grande eau et à tout moment le compartiment isolé 25.

Divers aménagements sont nécessaires pour permettre le montage des différents organes précités sur le châssis 50, notamment : interruption du réceptacle 3 en retrait de l'ouverture supérieure du compartiment 25 ; séparation de la goulotte 11 et du réceptacle 3, du vérin 16 et de la patte correspondante de la trappe 15, de la conduite 32 et de la paroi de l'enceinte 2 ; passage de la conduite 32 sous la poutre surmontant les portes 10, etc...

## Revendications

1. Collecteur de déchets spécifiques, notamment médicaux, comprenant une enceinte (2) en béton, un réceptacle intermédiaire (3), un conteneur amovible (4) de stockage des déchets comprenant un couvercle muni d'une trappe (18) normalement fermée, escamotable par coulissement, et des moyens (11 à 18) de mise en communication du réceptacle intermédiaire (3) alternativement avec l'extérieur du collecteur (1) et avec le conteneur (4), collecteur caractérisé en ce que le réceptacle intermédiaire (3) est disposé au-dessus du conteneur (4) de réception des déchets et est normalement fermé, en partie inférieure, par une trappe (15) escamotable par coulissement située au-dessus du couvercle du conteneur (4), ladite trappe (18) que comprend le couvercle du conteneur (4) étant escamotable selon la même direction que la trappe (15) du réceptacle intermédiaire (3), au moins l'une de ces trappes (15,18) étant munie de moyens (17) permettant la venue en prise des deux trappes (15,18) l'une avec l'autre lors de leur ouverture, de sorte que la trappe (15) du réceptacle intermédiaire (3), lorsqu'elle est déplacée dans le sens de son ouverture, déplace la trappe (18) du couvercle du conteneur (4) dans le sens de l'ouverture.

2. Collecteur selon la revendication 1, caractérisé en ce qu'il comprend un compartiment fermé (25), isolé thermiquement, dans lequel est placé le conteneur (4) de stockage des déchets, ainsi qu'une centrale (30) de production de froid à l'intérieur de ce compartiment (25), la trappe (15) du réceptacle intermédiaire (3) étant elle-même isolée thermiquement et assurant la fermeture de ce compartiment isolé (25).

3. Collecteur selon la revendication 1 ou la revendication 2, caractérisé en ce que ses différents organes, à l'exception du conteneur (4) et de l'éventuel compartiment isolé (25), ou une partie de ces organes seulement, sont montés sur un châssis coulissant (50) disposé au-dessus du conteneur (4) et de l'éventuel compartiment isolé (25), qui peut être au moins partiellement extrait du collecteur (1).

4. Collecteur selon l'une des revendications 1 à 3, caractérisé en ce que le réceptacle intermédiaire (3) comprend un télémètre (20) dont le signal est dirigé vers l'intérieur du conteneur (4).

5. Collecteur selon l'une des revendications 1 à 4, caractérisé en ce qu'il comprend une goulotte (11) de section carrée pour l'introduction des déchets, délimitée par quatre pans latéraux qui convergent vers l'intérieur du collecteur (1).

6. Collecteur selon l'une des revendications 1 à 5, caractérisé en ce qu'il comprend un système photo-électrique (14) placé au niveau de l'ouverture d'introduction des déchets, relié aux moyens de commande (13) de l'ouverture et de la fermeture de la porte coulissante (12) venant obturer le réceptacle intermédiaire (3) vis-à-vis de l'extérieur.

7. Collecteur selon l'une des revendications 1 à 6, caractérisé en ce qu'il comprend un lecteur (6) de carte muni d'un volet (21) de recouvrement de la fente d'introduction de la carte et de moyens de verrouillage de ce volet (21) en position abaissée au-dessus de la fente, ce volet (21) et ces moyens de verrouillage étant reliés à l'unité de commande de la porte (12) du réceptacle intermédiaire (3), l'ensemble permettant, lorsque le volet (21) est déplacé après que la carte ait été introduite dans le lecteur, le déclenchement de l'ouverture de la porte (12) du réceptacle intermédiaire (3) et le verrouillage du volet (21) en position abaissée tant que la porte (12) n'est pas refermée.

8. Collecteur selon l'une des revendications 1 à 7, caractérisé en ce qu'il comprend un ensemble (40 à 42) de pulvérisation de produit aseptique sur les déchets ou le sac contenant ces déchets, lorsque ceux-ci se trouvent dans le réceptacle intermédiaire (3).

9. Collecteur selon l'une des revendications 1 à 8, caractérisé en ce qu'il comprend une bavette frottant sur le dessus de la trappe escamotable (15).

10. Collecteur selon l'une des revendications 1 à 9, caractérisé en ce qu'il comprend un système de pesage encastré dans la trappe (15) du réceptacle intermédiaire (3).

11. Collecteur selon l'une des revendications 1 à 10, caractérisé en ce qu'il comprend un peigne ou similaire fixé à la trappe escamotable (18) du conteneur (4), permettant, lors du mouvement de cette trappe (18), de brasser les déchets afin de les répartir sur le fond du conteneur (4).

12. Collecteur selon l'une des revendications 1 à 11, caractérisé en ce qu'il comprend deux rampes (35) permettant de faciliter l'introduction et le retrait du conteneur (4), qui est monté sur roulettes (5), dans le collecteur (1) ou, le cas échéant, dans le compartiment isolé (25), ces rampes (35) étant basculables dans une position de repliage, dans laquelle elles portent contre la paroi latérale du conteneur (4) et maintiennent celui-ci en position de remplissage, sous le réceptacle intermédiaire (3).

13. Collecteur selon l'une des revendications 1 à 12, caractérisé en ce qu'il comprend un dispositif modulateur/démodulateur dit "modem", de transmission vers un centre de gestion des données recueillies à partir de la carte de l'utilisateur, ainsi que, le cas échéant, du télémètre (20) ou du dispositif de pesage.

14. Collecteur selon l'une des revendications 1 à 13, caractérisé en ce qu'il comprend une enceinte (2) en béton coulé en une seule pièce.

15. Collecteur selon l'une des revendications 1 à 14, caractérisé en ce qu'il comprend deux portes arrière (10) en acier, munies de serrures à trois points d'ancrage et d'un détecteur de leur position de fermeture.

16. Collecteur selon l'une des revendications 1 à 15, caractérisé en ce qu'il comprend une alarme sonore anti-vandalisme, à déclenchement en cas de violation des portes (10) assurant sa fermeture.

## Patentansprüche

1. Sammeleinrichtung für spezielle Abfälle, insbesondere medizinische Abfälle, umfassend eine Einfassung (2) aus Beton, einen Zwischenauffang (3), einen beweglichen Lagerbehälter (4) für Abfälle, der einen Deckel umfaßt, der mit einer normalerweise verschlossenen Klappe (18) versehen ist, die durch Verschieben einziehbar ist, und Mittel (11 bis 18) zum In-Verbindung-Setzen des Zwischenauffangs (3) alternativ mit der Umgebung der Sammeleinrichtung (1) und mit dem Behälter (4), wobei die Sammeleinrichtung **dadurch gekennzeichnet** ist, **daß** der Zwischenauffang (3) oberhalb des Behälters (4) zur Aufnahme des Abfalls angeordnet ist und normalerweise an der Unterseite durch eine Klappe (15) verschlossen ist, die durch Verschieben einziehbar ist und unter dem Deckel des Behälters (4) positioniert ist, wobei die Klappe (18), die der Deckel des Behälters (4) umfaßt, in dieselbe Richtung wie die Klappe (15) des Zwischenauffangs (3) einziehbar ist, und mindestens eine dieser Klappen (15, 18) mit Mitteln (17) versehen ist, die ein Mitnehmen der zwei Klappen (15, 18), der einen mit der anderen, und daher ihre Öffnung ermöglicht, derart, daß die Klappe (15) des Zwischenauffangs (3), wenn sie in die Richtung ihrer Öffnung verschoben wird, die Klappe (18) des Deckels des Behälters (4) in die Öffnungsrichtung verschiebt.

2. Sammeleinrichtung nach Anspruch 1, **dadurch gekennzeichnet, daß** sie einen geschlossenen thermisch isolierten Raum (25) umfaßt, in dem der Lagerbehälter (4) für den Abfall positioniert ist, sowie eine Zentrale (30) zur Erzeugung von Kälte in dem Inneren dieses Raumes (25), wobei die Klappe (15) des Zwischenauffangs (3) selber thermisch isoliert ist und den Verschluß dieses isolierten Raumes (25) gewährleistet.

3. Sammeleinrichtung nach Anspruch 1 oder 2, **dadurch gekennzeichnet, daß** die verschiedenen Bauteile außer dem Behälter (4) und eventuell der isolierte Raum (25) oder ein Teil dieser Bauteile, auf einem verschiebbaren Gestell (50) montiert sind, das unter dem Behälter (4) und eventuell dem isolierten Raum (25) angeordnet ist, das zumindest teilweise aus der Sammeleinrichtung (1) herausgezogen sein kann.

4. Sammeleinrichtung nach einem der Ansprüche 1 bis 3, **dadurch gekennzeichnet, daß** der Zwischenauffang (3) einen Abstandsmesser (20) umfaßt, dessen Signal durch den Innenraum des Behälters (4) geleitet wird.

5. Sammeleinrichtung nach einem der Ansprüche 1 bis 4, **dadurch gekennzeichnet, daß** sie eine Übergaberutsche (11) mit viereckigem Querschnitt für die Einführung von Abfall umfaßt, die durch Vierkantseiten begrenzt ist, die nach innen zu der Sammeleinrichtung (1) konvergieren.

6. Sammeleinrichtung nach einem der Ansprüche 1 bis 5, **dadurch gekennzeichnet, daß** sie ein fotoelektrisches System (14) umfaßt, das auf der Höhe der Öffnung zur Einführung von Abfall positioniert ist, das mit Steuermitteln (13) für das Öffnen und das Schließen der verschiebbaren Tür (12) verbunden ist, die den Zwischenauffang (3) gegenüber der Umgebung verschließt.

7. Sammeleinrichtung nach einem der Ansprüche 1 bis 6, **dadurch gekennzeichnet, daß** sie ein Kartenlesegerät (6) umfaßt, das mit einer Verschlußklappe (21) zur Abdeckung des Einführungsschlitzes der Karte und mit Mitteln zum Verriegeln dieser Verschlußklappe (21) in einer abgesenkten Position unter dem Schlitz versehen ist, wobei diese Verschlußklappe (21) und diese Mittel zum Verriegeln mit der Steuereinheit für die Tür (12) des Zwischenauffangs (3) verbunden sind, und der Aufbau, wenn die Verschlußklappe nachdem die Karte in das Lesegerät eingeführt wurde versetzt ist, die Auslösung der Öffnung der Tür (12) des Zwischenauffangs (3) und die Verriegelung der Verschlußklappe (21) in der abgesenkten Position ermöglicht, solange die Tür (12) nicht wieder geschlossen ist.

8. Sammeleinrichtung nach einem der Ansprüche 1 bis 7, **dadurch gekennzeichnet, daß** sie einen Aufbau (40 bis 42) zum Spritzen von aseptischen Stoffen auf den Abfall oder den Sack, der diesen Abfall enthält, der sich in dem Zwischenauffang (3) befindet, umfaßt.

9. Sammeleinrichtung nach einem der Ansprüche 1 bis 8, **dadurch gekennzeichnet, daß** sie einen griffigen Schmutzfänger auf der Unterseite der einziehbaren Klappe (15) umfaßt.

10. Sammeleinrichtung nach einem der Ansprüche 1 bis 9, **dadurch gekennzeichnet, daß** sie ein eingebautes Wiegesystem in der Klappe (15) des Zwischenauffangs (3) umfaßt.

11. Sammeleinrichtung nach einem der Ansprüche 1 bis 10, **dadurch gekennzeichnet, daß** sie einen Kamm oder ähnliches, der an der einziehbaren Klappe (18) des Behälters (4) fixiert ist, umfaßt, der durch die Bewegung dieser Klappe (18) ein Mischen der Abfälle durch das Verteilen auf dem Boden des Behälters (4) ermöglicht.

12. Sammeleinrichtung nach einem der Ansprüche 1 bis 11, **dadurch gekennzeichnet, daß** sie zwei Rampen (35) umfaßt, die es ermöglichen, die Einführung und das Herausziehen des Behälters (4) zu erleichtern, der auf Rollen (5) in der Sammeleinrichtung (1) oder nötigenfalls in dem isolierten Raum (25) montiert ist, wobei diese Rampen (35) in eine zusammengelegte Position klappbar sind, in der sie gegen die Seitenwände des Behälters (4) gestützt sind und in dieser zusammengelegten Position unter dem Zwischenauffang (3) verbleiben.

13. Sammeleinrichtung nach einem der Ansprüche 1 bis 12, **dadurch gekennzeichnet, daß** sie eine Vorrichtung zur Modulation/Demodulation, sprich "Modem" für die Übertragung von Kühlungsdaten von der Karte des Verwenders über ein Steuerungszentrum sowie nötigenfalls von Daten vom Abstandmesser (20) oder der Wiegevorrichtung umfaßt.

14. Sammeleinrichtung nach einem der Ansprüche 1 bis 13, **dadurch gekennzeichnet, daß** sie eine Einfassung (2) aus Beton umfaßt, die aus einem Stück gegossen ist.

15. Sammeleinrichtung nach einem der Ansprüche 1 bis 14, **dadurch gekennzeichnet, daß** sie zwei hintere Türen (10) aus Stahl umfaßt, die mit Schlössern an drei Verankerungspunkten und einem Detektor für ihre Schließposition versehen ist.

16. Sammeleinrichtung nach einem der Ansprüche 1 bis 15, **dadurch gekennzeichnet, daß** sie einen lauten Anti-Vandalismus-Alarm zur Auslösung im Fall der Beschädigung der Türen (10), die ihren Verschluß gewährleisten, umfaßt.

## Claims

1. A collector for specific waste, particularly medical waste, comprising a concrete enclosure (2), an intermediate receptacle (3), a removable waste storage container (4) comprising a cover provided with trap (18) which is normally closed and which can be retracted by sliding, and means (11 to 18) for placing the intermediate receptacle (3) in communication alternately with the outside of the collector (1) and with the container (4), said collector characterised in that the intermediate receptacle (3) is disposed above the container (4) for receiving waste and its lower part is normally closed by a trap (15) which is retractable by sliding and which is situated above the cover of the container (4), said trap (18), which comprises the cover of the container (4), being retractable in the same direction as the trap (15) of the intermediate receptacle (3), at least one of said traps (15, 18) being provided with means (17) permitting the two traps (15,18) to come into engagement with each other when they are opened in such a way that when the trap (15) of the intermediate receptacle (3) is displaced in the direction of opening thereof it displaces the trap (18) of the cover of the container (4) in the direction of opening.

2. A collector according to claim 1, characterised in that it comprises a closed compartment (25), which is thermally insulated and in which the waste storage container (4) is placed, as well as a central cooling installation (30) in the interior of said compartment (25), the trap (15) of the intermediate receptacle (3) being itself thermally insulated and effecting the closure of said insulated compartment (25).

3. A collector according to claim 1 or claim 2, characterised in that the various elements thereof, with the exception of the container (4) and of the optional insulated compartment (25), or part of said elements only, are mounted on a sliding frame (50) which is disposed above the container (4) and above the optional insulated compartment (25), and which can be withdrawn at least in part from the collector (1).

4. A collector according to any one of claims 1 to 3, characterised in that the intermediate receptacle (3) comprises a telemeter (20), the signal of which is directed towards the interior of the container (4).

5. A collector according to any one of claims 1 to 4, characterised in that it comprises a square section chute (11) for the introduction of waste, which chute is delimited by four side faces which converge towards the interior of the collector (1).

6. A collector according to any one of claims 1 to 5, characterised in that it comprises a photoelectric system (14) which is placed at the level of the waste introduction opening and which is connected to control means (13) for the opening and closing of the sliding door (12) which blanks off the intermediate receptacle (3) from the outside.

7. A collector according to any one of claims 1 to 6, characterised in that it comprises a card reader (6) which is provided with a shutter (21) for covering the card introduction slot and with means for locking said shutter (21) in a lowered position above the slot, said shutter (21) and said locking means being connected to the control unit for the door (12) of the intermediate receptacle (3), said system permitting, when the shutter (21) is displaced after the card has been introduced into the reader, the triggering of the opening of the door (12) of the intermediate receptacle (3) and the locking of the shutter (21) in a lowered position as long as the door (12) is not closed again.

8. A collector according to any one of claims 1 to 7, characterised in that it comprises a system (40 to 42) for spraying an aseptic product on to the waste or on to the bag containing said waste when these are situated in the intermediate receptacle (3).

9. A collector according to any one of claims 1 to 8, characterised in that it comprises a flap which rubs against the top of the retractable trap (15).

10. A collector according to any one of claims 1 to 9, characterised in that it comprises a weighing system installed in the trap (15) of the intermediate receptacle (3).

11. A collector according to any one of claims 1 to 10, characterised in that it comprises a rake or the like, which is fixed to the retractable trap (18) of the container (4) and which, when said trap (18) moves, enables the waste to be agitated in order to distribute it on the base of the container (4).

12. A collector according to any one of claims 1 to 11, characterised in that it comprises two ramps (35) which facilitate the introduction and withdrawal of the container (4), which is mounted on castors (5), into or from the collector (1) or into or from the insulated compartment (25) which is optionally present, said ramps (35) being tiltable into a folded-down position in which they bear against the side wall of the container (4) and maintain the latter in a filling position below the intermediate receptacle (3).

13. A collector according to any one of claims 1 to 12, characterised in that it comprises a modulator/demodulator device, termed a "modem", for transmitting data collected from the user's card, optionally as well as data collected from the telemeter (20) or from the weighing device, to a data management installation.

14. A collector according to any one of claims 1 to 13, characterised in that it comprises a concrete enclosure (2) which is cast in one piece.

15. A collector according to any one of claims 1 to 14, characterised in that it comprises two rear doors (10) made of steel, which are provided with locks at three anchoring points and with a detector for the closed position thereof.

16. A collector according to any one of claims 1 to 15, characterised in that it comprises an audible anti-vandalism alarm, which is triggered in the event of an unauthorised action on said doors (10) and ensures the closure thereof.
